# EUROPEAN PATENT SPECIFICATION

(11) **EP 4 089 414 B1**
(45) Date of publication and mention of the grant of the patent: **26.06.2024**
(21) Application number: 21859350.7
(22) Date of filing: 26.09.2021
(51) Int. Cl.: G01N 33/558, G01N 21/78, G01N 33/543

(54) **TEST PAPER TESTING AUXILIARY DEVICE**
HILFSPRÜFPAPIER-DETEKTIONSVORRICHTUNG
DISPOSITIF AUXILIAIRE D'ESSAI DE PAPIER RÉACTIF

(30) Priority: 25.03.2021 CN 202110319458; 28.07.2021 CN 202110859547
(43) Date of publication of application: 16.11.2022
(73) Proprietor: Jiangsu Bioperfectus Technologies Co., Ltd, Medical High-Tech Zone Taizhou Jiangsu 225300 (CN)
(72) Inventor: SHANG, Qianqian, Taizhou, Jiangsu 225300 (CN); JIA, Qianqian, Taizhou, Jiangsu 225300 (CN); JIANG, Xinyi, Taizhou, Jiangsu 225300 (CN); TAN, Linping, Taizhou, Jiangsu 225300 (CN); WANG, Xin, Taizhou, Jiangsu 225300 (CN); WEI, Fancheng, Taizhou, Jiangsu 225300 (CN); CHEN, Renyuan, Taizhou, Jiangsu 225300 (CN); JIN, Wei, Taizhou, Jiangsu 225300 (CN); LIU, Zhonghua, Taizhou, Jiangsu 225300 (CN); WANG, Guoqiang, Taizhou, Jiangsu 225300 (CN)
(74) Representative: Wilson Gunn
(86) International application number: PCT/CN2021/120553
(87) International publication number: WO 2022/198956

(56) References cited:
- EP-A1- 1 908 522
- WO-A2-2012/038820
- CN-A- 103 529 201
- CN-A- 108 051 579
- CN-A- 111 876 320
- CN-A- 113 075 396
- CN-U- 203 929 777
- CN-U- 211 576 971
- US-A1- 2007 128 070
- US-A1- 2015 198 592

## Description

### TECHNICAL FIELD

The present disclosure relates to the technical field of rapid diagnosis detection, and more specifically, relates to an auxiliary test paper detection device.

### BACKGROUND ART

Medical diagnosis of epidemics usually requires etiology-related examinations in laboratories, mainly including virus isolation, viral antigen, nucleic acid and antibody detection, and the like. The virus isolation is the "gold standard" for laboratory detection.

Virus isolation culture is to separate influenza viruses from respiratory tract specimens, but generally, it takes long time, has a high n culture difficulty and a low success rate. Viral antigen detection is a rapid detection method capable of adopting an immunofluorescence to detect mucosal epithelial cells in respiratory tract specimens (pharyngeal swabs, nasal swabs, nasopharyngeal swab or tracheal extracts), results can be generally obtained within 10-30 minutes, and the viral antigen detection is simple to operate and easy to use. Serological diagnosis is that the IgG antibody level by dynamic detection is increased by four times or above in the recovery period compared with that in the acute period, retrospective diagnostic significance is achieved, but double samples need to be collected for comparison. Virus nucleic acid detection has the best specificity and sensitivity, can quickly distinguish virus types and subtypes, can generally obtain results within 4-6 hours, but has high requirements for laboratory equipment and operator ability. Antibody detection can be used for retrospective investigation, but is not significant for early diagnosis of cases.

Comprehensively, existing viral antigen detection methods are easier to be recognized and accepted by the general public, pathogen detection needs to be carried out in hospital laboratories at present, but some problems exist in hospital treatment: specifically, firstly, the number of patients is large, and the waiting time of procedures such as queuing registration, waiting for diagnosis, examination, medicine taking and treatment is long and complex; secondly, the possibility of cross-infection between the patients is also caused by the contact of a plurality of patients in hospital; and thirdly, the economic burden is relatively high.

Therefore, a pathogen detection device which is safe and simple to operate and accurate in detection result is needed.

In addition, in current medical diagnosis products, detection of various to-be-detected sample (nasopharyngeal secretions, saliva, urine, blood, sweat and the like) usually requires pretreatment of the samples. Generally, a sample to be detected is pretreated (diluted or eluted) through an independent sample treatment device filled with a required buffer solution, so that a sample more suitable for detection is obtained. The sample is manually transferred from the sample treatment device into a sample adding hole provided with a test paper detection device, and after the reaction time is finished, a detection result is observed by naked eyes or read by virtue of an instrument. Whether the sample contains the detected target object or not is determined according to the detection result. Said method is a relatively mature and general method at present. However, in the detection process, the sample is transferred from the sample treatment device to the detection device, and multiple operation steps are needed, so that the detection process is complicated. In addition, generally, a pipettor or a plastic straight suction pipe is used for transferring the sample, so that the biological pollution of the environment is caused because the sample accidentally drops or excessive sample overflows from the sample adding hole due to the fact that dropping of the sample is not controlled in a detection area in the sample transferring process; and an inaccurate detection result may be caused by inaccurate transferred sample volume due to non-standard operation of an operator using the pipettor or the plastic straight pipette. At present, pathogen detection needs to be carried out in hospital laboratories at present, but a series of problems exist in hospital treatment: specifically, firstly, the number of patients is large, and the waiting time of procedures such as queuing registration, waiting for diagnosis, examination, medicine taking and treatment is long and complex; secondly, the possibility of cross-infection between the patients is also caused by the contact of a plurality of patients in hospital; and thirdly, the economic burden is relatively high.

Therefore, a detection device which can avoid environmental biological pollution and manual sample adding errors and is convenient to operate autonomously at home is also needed.

### SUMMARY

Aiming at the shortages existing in the prior art, the present disclosure aims to provide an auxiliary test paper detection device.

The above technical purpose of the present disclosure is realized through the following technical scheme.

An auxiliary test paper detection device is provided. The auxiliary test paper detection device includes a reagent cap and a test paper cassette. The reagent cap is configured for containing a detection reagent, a sealing member is arranged at an opening of the reagent cap, and the sealing member is opened or removed under action of external force; a test paper is arranged in the test paper cassette, a sample absorption section of the test paper is exposed, an observation window is formed in the test paper cassette, and a result display area of the test paper corresponds to the observation window. A connecting structure for connecting the test paper cassette and the reagent cap is provided; when the test paper cassette and the reagent cap are connected through the connecting structure, a closed space is formed by an inner cavity of the reagent cap, and the sample absorption section of the test paper extends into the inner cavity of the reagent cap.

In some embodiments, the test paper cassette includes an upper cover and a lower base which are in snap fit with each other, a gap for accommodating the test paper is formed between the upper cover and the lower base, and the upper cover and the lower base each are provided with positioning fasteners and first positioning blocking ribs. When the upper cover and the lower base are in snap fit, the positioning fasteners on the upper cover and the lower base are in snap fit with each other to form positioning lock, and the first positioning blocking ribs on the upper cover and the lower base cooperatively and tightly abut against the test paper.

In some embodiments, the connecting structure includes a connecting clamping sleeve and tail end fasteners, an inner periphery of an inner ring of the connecting clamping sleeve is fixedly sleeved on the test paper cassette, and an outer periphery of the inner ring of the connecting clamping sleeve is sleeved with the reagent cap and in interference fit with the reagent cap. The tail end fasteners are elastic, and the tail end fasteners are respectively arranged on the upper cover and the lower base at an end of the test paper cassette, fixedly sleeved with the connecting clamping sleeve. When the connecting clamping sleeve is sleeved to a predetermined station, the inner ring of the connecting clamping sleeve is in snap fit with the tail end fasteners on the upper cover and the lower base.

In some embodiments, the test paper cassette includes an upper cover and a lower base, a positioning hole is formed in the upper cover, a positioning convex block is arranged on the lower base, the positioning convex block is embedded in the positioning hole and in interference fit with the positioning holes; a gap for accommodating the test paper is formed between the upper cover and the lower base; and an end of the test paper cassette, from which the sample absorption section of the test paper extends out, has a shape adapted to an inner wall of the reagent cap.

In some embodiments, the connecting structure includes two handles, the two handles are respectively and movably arranged at two sides of the end of the test paper cassette, from which the sample absorption section of the test paper extends out, and main bodies of the two handles extend outwards in an insertion direction of the reagent cap.

In some embodiments, the test paper cassette includes an upper cover and a lower base which are in snap fit with each other, a gap for accommodating the test paper is formed between the upper cover and the lower base, positioning mounting holes are formed in the upper cover, and mounting columns are arranged on the lower base. When the upper cover and the lower base are in snap fit with each other, the mounting columns are inserted into the positioning mounting holes and in interference fit with the positioning mounting holes.

In some embodiments, the connecting structure includes an insertion member, an outer contour of the insertion member corresponds to a side wall of an inner cavity of the reagent cap, and the insertion member is in interference fit with the side wall of the inner cavity of the reagent cap.

In some embodiments, the test paper cassette includes an upper cover and a lower base which are in snap fit with each other, a gap for accommodating the test paper is formed between the upper cover and the lower base, positioning fasteners are arranged on the upper cover and the lower base, and second positioning blocking ribs are arranged on the upper cover and the lower base. When the upper cover is in snap fit with the lower base, the positioning fasteners on the upper cover and the lower base are in snap fit together and form a positioning lock, and the second positioning blocking ribs on the upper cover and the lower base cooperatively and tightly abut against the test paper.

In some embodiments, the connecting structure includes an elastic sealing member, the elastic sealing member is arranged between the test paper and the test paper cassette, and the elastic sealing member is located at an end of the test pater cassette where the sample absorption section of the test paper is located. The end of the test pater cassette where the sample absorption section of the test paper is located has a shape adapted to an outer wall of the reagent cap, and an outer contour of the elastic sealing member is adapted to the inner cavity of the reagent cap.

In some embodiments, the elastic sealing member includes inner rubber plugs and an outer rubber plug, the inner rubber plugs include a pair of split rubber plugs which are connected with each other in an insertion manner, the inner rubber plugs are sleeved on the test paper, one end of the outer rubber plug is sleeved on the inner rubber plugs to form secondary sealing, and another end of the outer rubber plug is inserted into the inner cavity of the reagent cap to form sealing.

The present disclosure aims to solve the problem that people can conveniently perform pathogen detection in families after influenza A test strip and influenza B test strip are combined, whether body discomfort is caused by influenza virus or not can be preliminarily judged, and whether the body discomfort is caused by influenza A virus or influenza B virus can be further distinguished.

Compared with the prior art, the present disclosure has the following beneficial effects.

Firstly, liquid is sub-packaged in the reagent cap in advance, and the test paper cassette and the reagent cap are connected in an insertion mode, which is simple in operation and facilitates to reduce manual operation and result errors caused by manual sample adding.

Secondly, a closed reaction device is formed in the reagent cap, so that biological pollution is reduced when the present disclosure is used in a household environment, thereby protecting the user and the household environment.

Thirdly, by self-testing pathogens at home, cross infection caused in hospitals is avoided, and the waste of manpower, material resources and financial resources caused by queuing and registering in hospitals is avoided;

Fourthly, different types of influenza pathogens can be detected by using different test paper, and other similar respiratory pathogens can also be detected, so that the applicability of the auxiliary test paper detection device can be improved.

Fifthly, the whole detection device is a closed reaction device, so that biological pollution is reduced when the detection device is used in a household environment, thereby protecting the user and the household environment.

Sixthly, by self-testing pathogens at home, cross infection caused in hospitals is avoided, and the waste of manpower, material resources and financial resources caused by queuing and registering in hospitals is avoided;

The detection device disclosed by the present disclosure is wide in adaptability, and can be matched with test paper of other items for testing (such as other respiratory pathogens, hormones and the like), so that not only influenza pathogens can be detected at home, but also other similar respiratory pathogens, cancer targets and hormone targets can be detected, thereby providing further convenience to people.

### BRIEF DESCRIPTION OF THE DRAWINGS

To describe the technical scheme in the embodiments of the present disclosure or in the prior art more clearly, the following briefly introduces the accompanying drawings required for describing the embodiments. Apparently, the accompanying drawings in the following description show merely some embodiments of the present disclosure, and those skilled in the art may still derive other accompanying drawings from these accompanying drawings without creative efforts.
FIG. 1 is a cross-sectional schematic diagram of an overall structure of an auxiliary test paper detection device in Embodiment 1 of the present disclosure;
FIG. 2 is an external schematic diagram of the overall structure of the auxiliary test paper detection device in Embodiment 1 of the present disclosure;
FIG. 3 is an explosive schematic diagram of the overall structure of the auxiliary test paper detection device in Embodiment 1 of the present disclosure;
FIG. 4 is a schematic diagram of an overall structure of a reagent cap in Embodiment 1 of the present disclosure;
FIG. 5 is a cross-sectional schematic diagram of an overall structure of an auxiliary test paper detection device in Embodiment 2 of the present disclosure;
FIG. 6 is an external schematic diagram of the overall structure of the auxiliary test paper detection device in Embodiment 2 of the present disclosure;
FIG. 7 is an explosive schematic diagram of the overall structure of the auxiliary test paper detection device in Embodiment 2 of the present disclosure;
FIG. 8 is a cross-sectional schematic diagram of an overall structure of an auxiliary test paper detection device in Embodiment 3 of the present disclosure;
FIG. 9 is an external schematic diagram of the overall structure of the auxiliary test paper detection device in Embodiment 3 of the present disclosure;
FIG. 10 is an explosive schematic diagram of the overall structure of the auxiliary test paper detection device in Embodiment 3 of the present disclosure;
FIG. 11 is a cross-sectional schematic diagram of an overall structure of an auxiliary test paper detection device in Embodiment 4 of the present disclosure; and
FIG. 12 is an explosive schematic diagram of the overall structure of the auxiliary test paper detection device in Embodiment 4 of the present disclosure.

List of Reference signs: 1' lower base; 2' upper cover; 21' ventilated window; 22' observation window; 3' test paper; 4' reagent cap; 5' positioning fastener; 51' first positioning blocking rib; 52' connecting clamping sleeve; 53' tail end fastener; 6 positioning hole; 61' positioning convex block; 62' handle; 7' positioning mounting hole; 71' mounting column; 72' insertion member; 8' positioning fastener; 81' second positioning blocking rib; 82' lower inner rubber plug; 83' upper inner rubber plug; and 84' outer rubber plug.

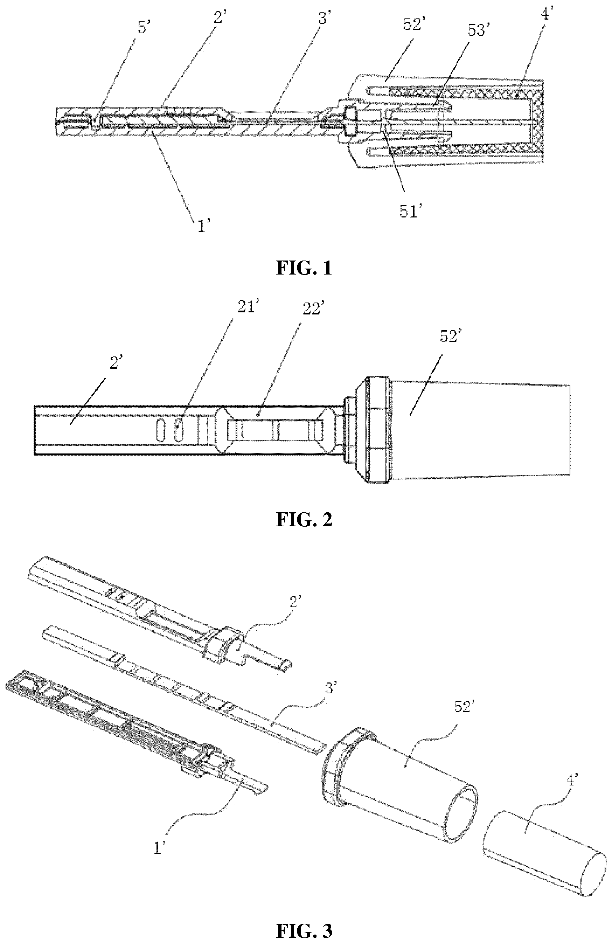

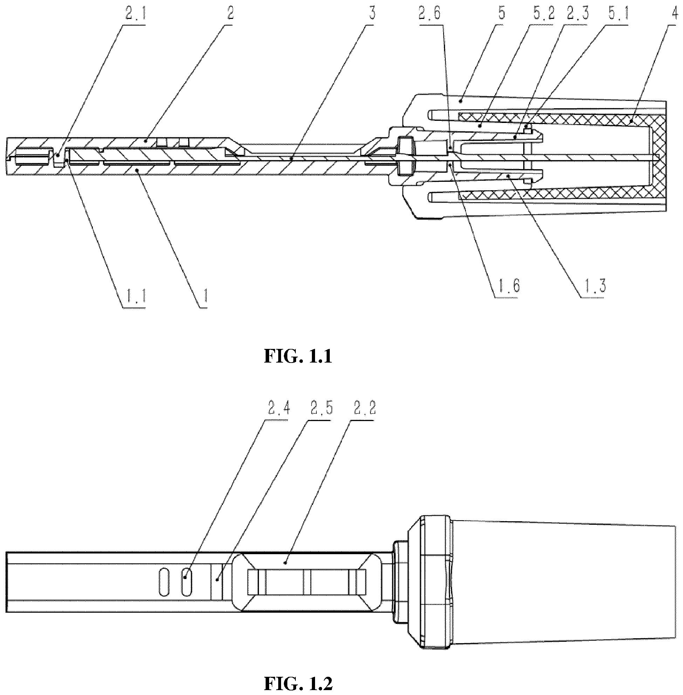

FIG. 1.1 is a cross-sectional structural schematic diagram of Embodiment 5;
FIG. 1.2 is a structural schematic diagram in top view of Embodiment 5;

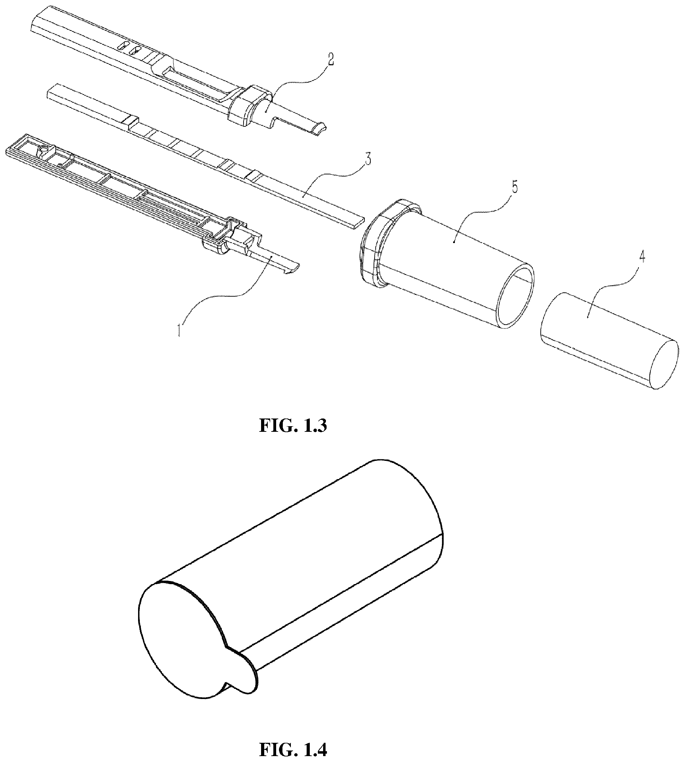

FIG. 1.3 is an assembly structural schematic diagram of Embodiment 5; and
FIG. 1.4 is a stereoscopic schematic diagram of a reagent bearing device in Embodiment 5.

List of reference signs: 1 lower base; 1.1 first positioning fastener; 1.3 lower base tail end fastener; 1.6 lower base positioning blocking rib; 2 upper cover; 2.1 second positioning fastener; 2.3 upper cover tail end fastener; 2.2 reaction window; 2.6 upper cover positioning blocking rib; 3 test paper; 4 reagent bearing device; 5 clamping sleeve; 5.1 clamping-sleeve inner ring tail end; and 5.2 clamping- sleeve inner ring middle section.

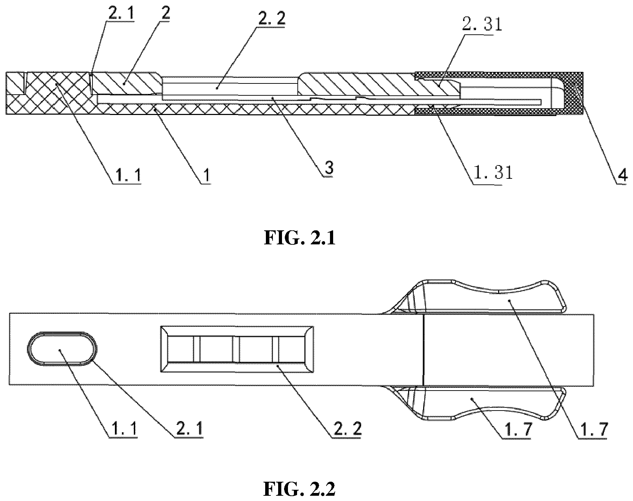

FIG. 2.1 is a cross-sectional structural schematic diagram of Embodiment 6;
FIG. 2.2 is a structural schematic diagram in top view of Embodiment 6;

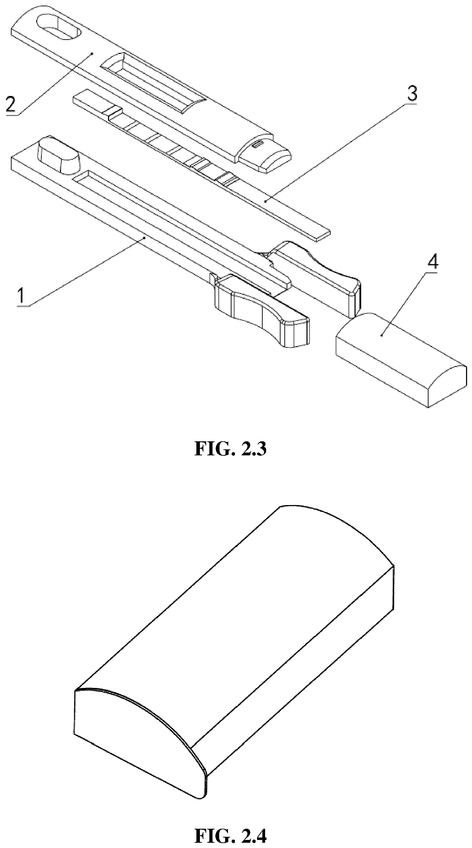

FIG. 2.3 is an assembly structural schematic diagram of Embodiment 6; and
FIG. 2.4 is a stereoscopic schematic diagram of a reagent bearing device in Embodiment 6.

List of reference signs: 1 lower base; 1.1 first positioning fastener; 1.31 lower base tail end; 1.7 lower base handle; 2 upper cover; 2.1 second positioning fastener; 2.31 upper cover tail end; 2.2 reaction window; 3 test paper; and 4 reagent bearing device.

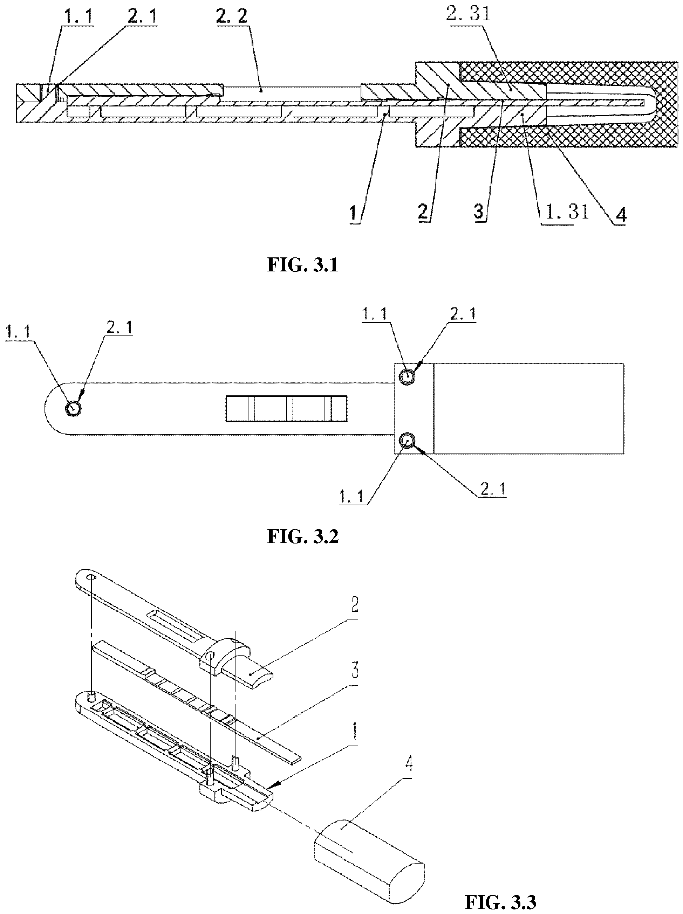

FIG. 3.1 is a cross-sectional structural schematic diagram of Embodiment 7;
FIG. 3.2 is a structural schematic diagram in top view of Embodiment 7;
FIG. 3.3 is an assembly structural schematic diagram of Embodiment 7; and

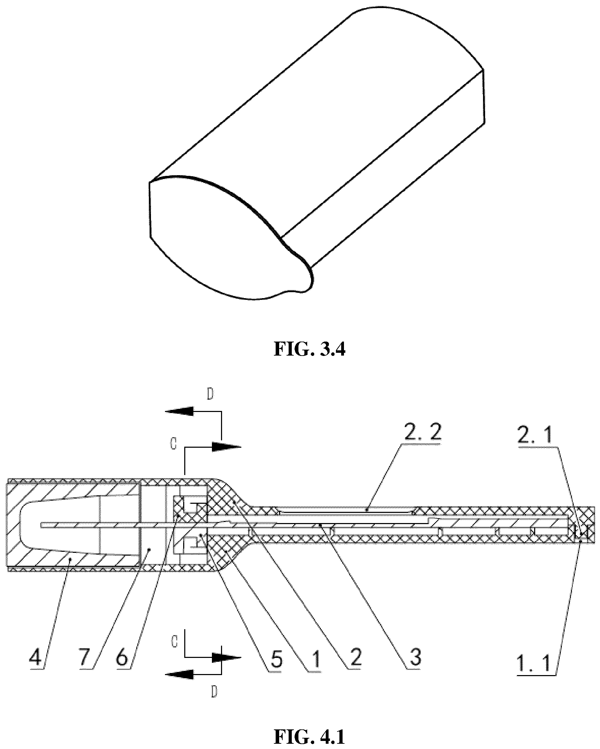

FIG. 3.4 is a stereoscopic schematic diagram of a reagent bearing device in Embodiment 7.

List of reference signs: 1 lower base; 1.1 first positioning fastener; 1.31 lower base tail end; 2 upper cover; 2.1 second positioning fastener; 2.2 reaction window; 2.31 upper cover tail end; 3 test paper; and 4 reagent bearing device.

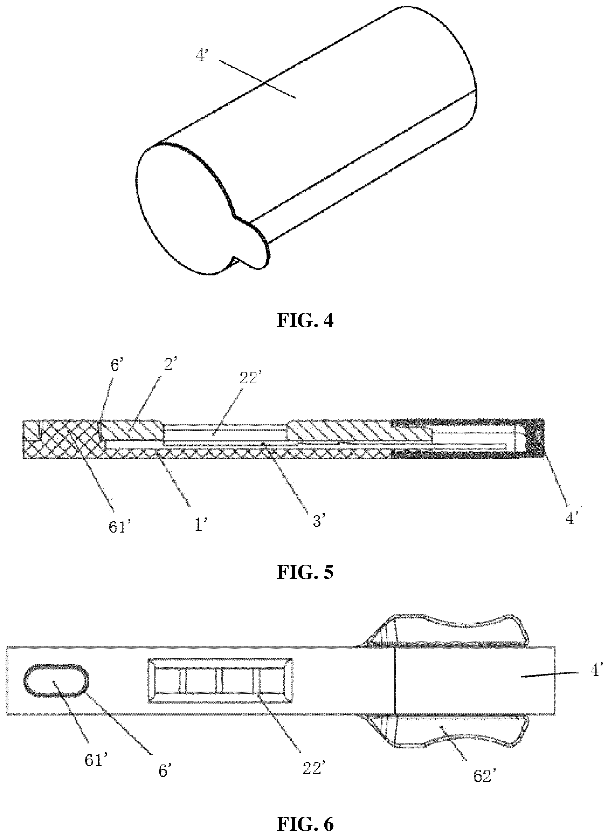

FIG. 4.1 is a cross-sectional structural schematic diagram of Embodiment 8;

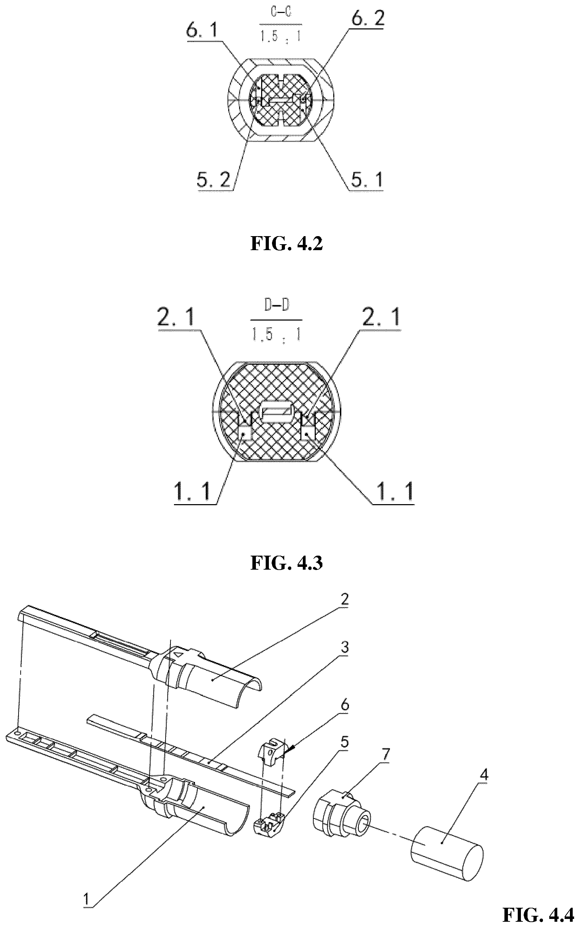

FIG. 4.2 is a cross-sectional structural schematic diagram along the C-C direction in FIG. 4.1;
FIG. 4.3 is a cross-sectional structural schematic diagram along the D-D direction in FIG. 4.2;
FIG. 4.4 is an assembly structural schematic diagram of Embodiment 8; and

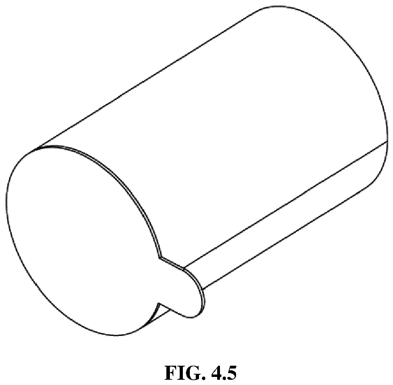

FIG. 4.5 is a stereoscopic schematic diagram of a reagent bearing device in Embodiment 8.

List of reference signs: 1 lower base; 1.1 first positioning fastener; 2 upper cover; 2.1 second positioning fastener; 2.2 reaction window; 3 test paper; 4 reagent bearing device; 51 lower inner rubber plug; 5.1 lower-inner-rubber-plug groove; 5.2 lower-inner-rubber-plug rivet; 6 upper inner rubber plug; 6.1 upper-inner-rubber-plug groove; 6.2 upper-inner-rubber-plug rivet; and 7 outer rubber plug.

### DETAILED DESCRIPTION OF THE EMBODIMENTS

The following clearly and completely describes the technical scheme in the embodiments of the present disclosure with reference to the accompanying drawings in the embodiments of the present disclosure. Apparently, the described embodiments are merely a part rather than all of the embodiments of the present disclosure. Based on the embodiments in the present disclosure, all other embodiments obtained by the ordinary technical staff in the art under the premise of without contributing creative labor belong to the scope protected by the present disclosure.

The present disclosure aims to provide an auxiliary test paper detection device to solve the problems in the prior art.

To make the foregoing objective, features and advantages of the present disclosure clearer and more comprehensible, the present disclosure is further described in detail below with reference to the accompanying drawings and specific embodiments.

### Embodiment 1

As shown in FIG. 1 to FIG. 3, the present disclosure provides an auxiliary test paper detection device. The auxiliary test paper detection device includes a reagent cap 4' and a test paper cassette. The reagent cap 4' is used for containing a detection reagent. Test paper 3' is arranged in the test paper cassette, a sample absorption section of the test paper 3' extends out from one end of the test paper cassette and is exposed, an observation window 22' is formed in the test paper cassette, and a result display area of the test paper 3' corresponds to the observation window 22'. A connecting structure is connected between the test paper cassette and the reagent cap 4', and when the test paper cassette and the reagent cap 4' are connected through the connecting structure, a closed space is formed in an inner cavity of the reagent cap 4', and the sample absorption section of the test paper 3' extends into the inner cavity of the reagent cap 4'.

The test paper cassette includes an upper cover 2' and a lower base 1' which are in snap fit with each other, the upper cover 2' and the lower base 1' are both of strip structures, a gap for accommodating test paper 3' is formed between the upper cover 2' and the lower base 1'. The test paper 3' is fixed through snap fit of the upper cover 2' and the lower base 1'. An observation window 22' is formed in the upper cover 2', a result display area of the test paper 3' corresponds to the observation window 22', and the sample absorption section of the test paper 3' extends out of the length end of the test paper cassette.

Positioning fasteners 5' and first positioning blocking ribs 51' are integrally formed on the upper cover 2' and the lower base 1'. The upper cover 2' and the lower base 1' are assembled into one piece through the two positioning fasteners 5', and the two positioning fasteners 5' are located on the end of the test paper cassette away from the sample absorption section of the test paper 3'.

A plurality of first positioning blocking ribs 51' are integrally formed in the middle of the upper cover 2' and the middle of the lower base 1' at intervals, each first positioning blocking rib 51' abuts against the test paper 3', the test paper 3' is clamped and fixed through the first positioning blocking ribs 51' so as to prevent the test paper 3' from sliding inwards and outwards. When the upper cover 2' and the lower base 1' are in snap fit, interference fit is formed through the two positioning fasteners 5', so that the upper cover 2' and the lower base 1' are completely sealed.

The connecting structure includes a connecting clamping sleeve 52' and tail end fasteners 53', the tail end fasteners 53' are elastic, and one tail end fasteners 53' are integrally formed respectively on the upper cover 2' and the lower base 1' at the end of the test paper cassette from which the sample absorption section of the test paper 3' extends out. Inverted hook structures are formed at the ends of the two tail end fasteners 53' away from the test paper cassette. The connecting clamping sleeve 52' is of a hollow barrel shape, the inner diameter of the connecting clamping sleeve52' is gradually increased from one end to the other end, an inner ring is integrally formed in the connecting clamping sleeve 52', the inner ring is fixedly connected with the end, with the largest inner diameter, of the connecting clamping sleeve 52', and the inner ring and the inner wall of the connecting clamping sleeve 52' are arranged at intervals.

The connecting clamping sleeve 52' is sleeved with the test paper cassette from the end, with the sample absorption section of the test paper 3', of the test paper cassette until the test paper cassette 52' cannot be pushed, and through the cooperation of the two tail end fasteners 53' and the inner ring of the connecting clamping sleeve52', when the connecting clamping sleeve 52' is sleeved to a predetermined station, the inner ring of the connecting clamping sleeve 52' respectively abuts against the inverted hook structures of the two tail end buckles 53', so that it is guaranteed that the upper cover 2' and the lower base 1' are completely sealed.

As shown in FIG. 4', the reagent cap 4' is filled with the detection reagent, and the reagent cap 4' is sealed with a sealing film or sealed by a cap, so that it is guaranteed that liquid in the device does not leak before use.

As shown in FIG. 1 and FIG. 3', when detection is needed, a sealing member of the reagent cap 4' is firstly opened, the test paper cassette is vertically inserted into the reagent cap 4' with the sample absorption section of the test paper 3' downwards until the test paper cassette is not pushed. The reagent cap and the connecting clamping sleeve are in interference fit through cooperation of the large-to-small structure of the inner ring of the reagent cap 4' and the small-to-large structure of a middle section of the inner ring of the connecting clamping sleeve, which ensures that when the reagent cap 4' is horizontally placed, liquid in the reagent cap 4' cannot leak laterally. In addition, the sample absorption section of the test paper 3' can be immersed into the liquid in the reagent cap 4', the test paper 3' is tightly clamped through the two first positioning blocking ribs 51', the test paper 3' can be firmly fixed; meanwhile, the liquid in the reagent cap 4' is prevented from rushing to the test paper 3' at the moment of horizontal placement. In 10-15 minutes of a chromatographic reaction, the liquid in the reagent cap 4' can be gradually chromatographed from the sample absorption end of the test paper 3' to the result display area of the test paper 3' and then to the top end of the test paper 3' until the chromatographic reaction is finished. Finally, a value is read by inserting the device into the instrument or by observing the observation window 22' through the naked eyes.

As shown in FIG. 1 to FIG. 3, a ventilated window 21' is further formed in the upper cover 2', after 10-15 minutes of the reaction is finished, the instrument obtains a signal value through scanning and interpreting the area of the observation window 22', analyzes the signal value and converts the signal value into a detection result, and provides whether pathogen infection exists or not, or whether pathogen infection exists or not is given out by observing the line color developing intensity of the test paper 3' in the area of the observation window 22' in combination with a result interpretation standard.

The reagent cap 4' serves as a sample pre-processing device or a sample processing device, and processed sample types include not only swab samples, but also samples such as feces, blood and urine. Furthermore, the auxiliary test paper detection device in the present application can be used for non-quantitative detection and for quantitative detection.

### Embodiment 2

As shown in FIG. 5 to FIG. 7, the present disclosure provides an auxiliary test paper detection device. The auxiliary test paper detection device includes a reagent cap 4' and a test paper cassette. The reagent cap 4' is used for containing a detection reagent. Test paper 3' is fixed in the test paper cassette, a sample absorption section of the test paper 3' extends out from one end of the test paper cassette and is exposed, an observation window 22' is formed in the test paper cassette, and a result display area of the test paper 3' corresponds to the observation window 22'. A connecting structure is connected between the test paper cassette and the reagent cap 4', and when the test paper cassette and the reagent cap 4' are connected through the connecting structure, a closed space is formed in an inner cavity of the reagent cap 4', and the sample absorption section of the test paper 3' extends into the inner cavity of the reagent cap 4'.

The test paper cassette includes an upper cover 2' and a lower base 1' which are in snap fit with each other. The upper cover 2' and the lower base 1' are both of strip structures, a gap for accommodating test paper 3' is formed between the upper cover 2' and the lower base 1', and the test paper 3' is fixed through the snap fit cooperation of the upper cover 2' and the lower base 1'. An observation window 22' is formed in the upper cover 2', a result display area of the test paper 3' corresponds to the observation window 22', and the sample absorption section of the test paper 3' extends out of the length end of the test paper cassette.

A positioning hole 6' is formed in the upper cover 2', the positioning hole 6' is formed in the end, away from the sample absorption section of the test paper 3', of the test paper cassette, a positioning convex block 61' is integrally formed on the lower base 1' and located at the end, away from the sample absorption section of the test paper 3', of the test paper cassette. The positioning hole 6' and the positioning convex block 61' are arranged correspondingly. When the upper cover 2' and the lower base 1' are in snap fit, the positioning convex block 61' is embedded into the positioning hole 6' and is in interference fit with the positioning hole 6', so that the upper cover 2' and the lower base 1' are tightly combined, thereby improving the mounting stability of the test paper 3' in the test paper cassette. And the shape of the end of the test paper cassette from which the sample absorption section of the test paper 3' extends out, is adapted to the inner wall of the reagent cap 4'.

The connecting structure includes two handles 62'. The two handles 62' are symmetrically hinged to the lower base 1' at two sides of the test paper cassette in the width direction respectively, and the two handles 62' are both located on the side of the test paper cassette close to the sample absorption section of the test paper 3'. And main bodies of the two handles 62' extend outwards along the plug-in direction of the reagent cap' 4, and the two handles 62' are arc-shaped.

A certain amount of liquid can be sub-packaged in the reagent cap 4' in advance, and the reagent cap 4' is sealed with a sealing film or sealed by a cap, so that it is guaranteed that liquid in the device does not leak before use.

As shown in FIG. 5 to FIG. 7, when detection is needed, firstly, a sealing member of the reagent cap 4' is opened, the test paper cassette is vertically inserted into the reagent cap 4' with the sample absorption section of the test paper 3' downwards until the test paper cassette is not pushed. The tail end of the upper cover 2' and the tail end of the lower base 1' are assembled to form a insertion portion which is gradually enlarged from right to left and is basically consistent with the inner ring of the inner cavity of the reagent cap 4' in shape, and the insertion portion and the reagent cap 4' are in interference fit. In addition, two handles 62' on the lower base 1' can simultaneously perform secondary extrusion sealing on the reagent cap 4', so that liquid in the reagent cap 4' can be doubly guaranteed not to overflow in the reaction process, thereby avoiding biological pollution.

In addition, the arc-shaped design of the handle 62' at the lower base 1' conforms to manual function science and is convenient for people to grasp manually. The sample absorption section of the test paper 3' can be immersed into the liquid in the reagent cap 4', and the test paper 3' is stably mounted in the test paper cassette through the cooperation of the positioning hole 6' and the positioning convex block 61', so that the liquid in the reagent cap 4' is prevented from rushing to the test paper 3' at the moment of horizontal placement. Thus, in 10-15 minutes of a chromatographic reaction, the liquid in the reagent cap 4' can be gradually chromatographed from the sample absorption end of the test paper 3' to the result display area of the test paper 3' and then to the top end of the test paper 3' until the chromatographic reaction is finished. Finally, a value is read through inserting the device into the instrument or by observing the observation window 22' through naked eyes.

After 10-15 minutes of the reaction is finished, the instrument obtains a signal value after scanning and interpreting the area of the observation window 22', analyzes the signal value and converts the signal value into a detection result, and gives whether pathogen infection exists or not, or whether pathogen infection exists or not is given out by observing the line color developing intensity of the test paper 3' in the area of the observation window 22' in combination with a result interpretation standard.

The reagent cap 4' serves as a sample pre-processing device or a sample processing device, and processed sample types include not only swab samples, but also samples such as feces, blood and urine. Furthermore, the auxiliary test paper detection device in the present application can be used for non-quantitative detection and also can be used for quantitative detection.

### Embodiment 3

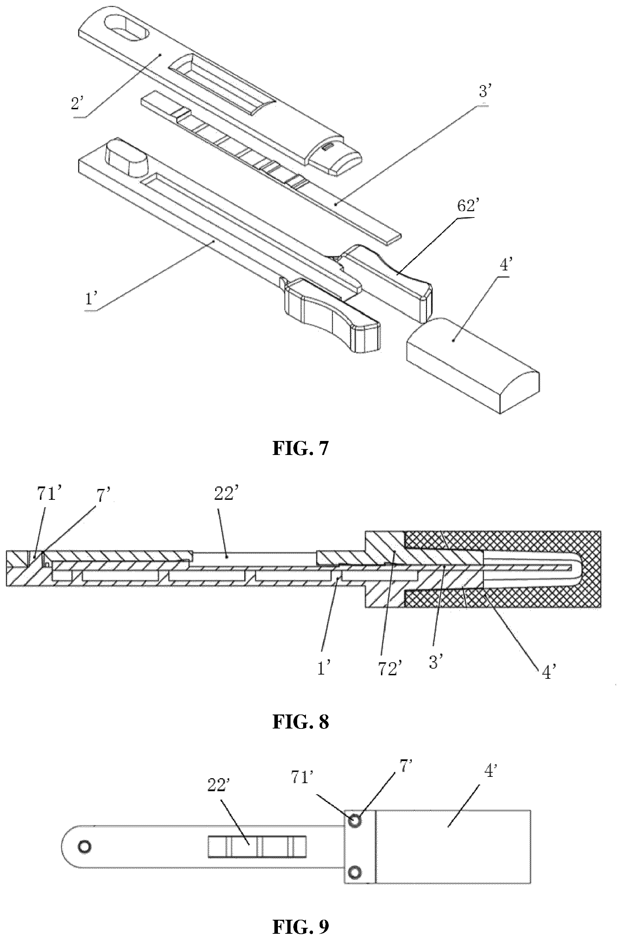

As shown in FIG. 8 to FIG. 10, the present disclosure provides an auxiliary test paper detection device. The auxiliary test paper detection device includes a reagent cap 4' and a test paper cassette. The reagent cap 4' is used for containing a detection reagent. Test paper 3' is fixed in the test paper cassette, a sample absorption section of the test paper 3' extends out from one end of the test paper cassette and is exposed, an observation window 22' is formed in the test paper cassette. A result display area of the test paper 3' corresponds to the observation window 22'. A connecting structure is provided between the test paper cassette and the reagent cap 4'. When the test paper cassette and the reagent cap 4' are connected through the connecting structure, a closed space is formed in an inner cavity of the reagent cap 4', and the sample absorption section of the test paper 3' extends into the inner cavity of the reagent cap 4'.

The test paper cassette includes an upper cover 2' and a lower base 1' which are in snap fit with each other. The upper cover 2' and the lower base 1' are both of strip structures. A gap for accommodating test paper 3' is formed between the upper cover 2' and the lower base 1', and the test paper 3' is fixed through the snap fit cooperation of the upper cover 2' and the lower base 1'. An observation window 22' is formed in the upper cover 2', a result display area of the test paper 3' corresponds to the observation window 22', and the sample absorption section of the test paper 3' extends out of the length end of the test paper cassette.

Positioning mounting holes 7' are formed in the upper cover 2', one positioning mounting hole 7' is formed in the middle of the end, away from the sample absorption section of the test paper 3', of the upper cover 2', and one positioning mounting hole 7' is formed in each of the two sides of the end, close to the sample absorption section of the test paper 3', of the upper cover 2'. Mounting columns 71' are integrally formed on the lower base 1'. The number and positions of the mounting columns 71' correspond to those of the positioning mounting holes 7' in the upper cover 2'. During mounting, the test paper 3' is placed in a gap formed through the cooperation of the upper cover 2' and the lower base 1'. Each mounting column 71' is embedded into the corresponding positioning mounting hole 7' and is in interference fit with the corresponding positioning mounting hole 7', so that the upper cover 2' and the lower bottom 1' are matched to press the test paper 3'.

The connecting structure includes an insertion member 72'. The insertion member 72' is formed through the cooperation of the ends, close to the sample absorption section of the test paper 3', of the upper cover 2' and the lower base 1'. The outer contour of the insertion member 72' corresponds to the inner ring of the inner cavity of the reagent cap 4', and the insertion member 72' is in interference fit with the side wall of the inner cavity of the reagent cap 4'.

A certain amount of liquid can be sub-packaged in the reagent cap 4' in advance, and the reagent cap 4' is sealed with a sealing film or sealed by a cap, so that it is guaranteed that liquid in the device does not leak before use.

As shown in FIG. 8 to FIG. 10, when detection is needed, a sealing member of the reagent cap 4' is firstly opened, the test paper cassette is vertically inserted into the reagent cap 4' with the sample absorption section of the test paper 3' downwards until the test paper cassette is not pushed. The tail end of the upper cover 2' and the tail end of the lower base 1' are matched to form the insertion member 72', a size of the insertion member 72' is gradually increased from right to left, the shape of the insertion member 72' is basically consistent with the large-to-small inner ring of the inner cavity of the reagent cap 4'. The insertion member72' and the reagent cap 4' are in interference fit, so that it is guaranteed that liquid in the reagent cap 4' cannot overflow in the reaction process, thereby avoiding biological pollution.

The sample absorption section of the test paper 3' can be completely immersed into the liquid in the reagent cap 4', the test paper 3' is tightly clamped through the cooperation of the tail ends of the upper cover 2' and the lower base 1', thereby preventing the liquid in the reagent cap 4' from rushing to the test paper 3' at the moment of horizontal placement. In 10-15 minutes of a chromatographic reaction, the liquid in the reagent cap 4' can be gradually chromatographed from the sample absorption end of the test paper 3' to the result display area of the test paper 3' and then to the top end of the test paper 3' until the chromatographic reaction is finished. Finally, a value is read by inserting the device into the instrument or by observing the observation window 22' through naked eyes.

After 10-15 minutes of the reaction is finished, the instrument obtains a signal value after scanning and interpreting the area of the observation window 22', analyzes the signal value and converts the signal value into a detection result and gives whether pathogen infection exists or not, or whether pathogen infection exists or not is given out by observing the line color developing intensity of the test paper 3' in the area of the observation window 22' in combination with a result interpretation standard.

The reagent cap 4' serves as a sample pre-processing device or a sample processing device, and processed sample types include not only swab samples, but also samples such as feces, blood and urine. Furthermore, the auxiliary test paper detection device in the present application can be used for non-quantitative detection and also can be used for quantitative detection.

### Embodiment 4

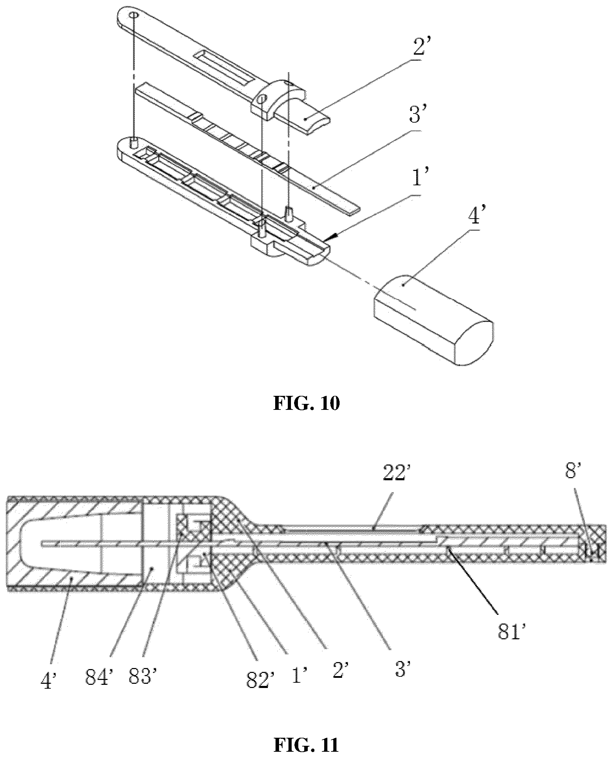

As shown in FIG. 11 to FIG. 12, the present disclosure provides an auxiliary test paper detection device. The auxiliary test paper detection device includes a reagent cap 4' and a test paper cassette. The reagent cap 4' is used for containing a detection reagent. Test paper 3' is fixed in the test paper cassette, a sample absorption section of the test paper 3' extends out from one end of the test paper cassette and is exposed. An observation window 22' is formed in the test paper cassette. A result display area of the test paper 3' corresponds to the observation window 22'. A connecting structure is connected between the test paper cassette and the reagent cap 4'. When the test paper cassette and the reagent cap 4' are connected through the connecting structure, a closed space is formed in an inner cavity of the reagent cap 4', and the sample absorption section of the test paper 3' extends into the inner cavity of the reagent cap 4'.

The test paper cassette includes an upper cover 2' and a lower base 1' which are in snap fit with each other. The upper cover 2' and the lower base 1' are both of strip structures. A gap for accommodating test paper 3' is formed between the upper cover 2' and the lower base 1'. The test paper 3' is fixed through the snap fit cooperation of the upper cover 2' and the lower base 1'. An observation window 22' is formed in the upper cover 2', a result display area of the test paper 3' corresponds to the observation window 22'. The sample absorption section of the test paper 3' extends out from the length end of the test paper cassette.

Positioning fasteners 8 are integrally formed on the upper cover 2' and the lower base 1'. The two positioning fasteners 8 are both located on the end of the test paper cassette away from the sample absorption section of the test paper 3', and the two positioning fasteners 8 are in snap fit with each other. A plurality of second positioning blocking ribs 81' are integrally formed on the upper cover 2' and the lower base 1'. The second positioning blocking ribs 81' are integrally formed on the upper cover 2' and the lower base 1' at equal intervals in the length direction of the test paper cassette.

When the upper cover 2' and the lower base 1' are in snap fit, the positioning fastener 8 on the upper cover 2' is in snap fit with the positioning fastener 8 on the lower base 1'. The second positioning blocking rib 81' on the upper cover 2' and the positioning blocking rib 81' on the lower base 1' abut against the test paper 3' tightly, so that the test paper 3' is stably mounted in the test paper cassette

The connecting structure includes an elastic sealing member. The elastic sealing member is mounted at the end of the test paper cassette close to the sample absorption section of the test paper 3'. The elastic sealing member is mounted between the test paper 3' and the test paper cassette and includes an inner rubber plug and an outer rubber plug 84'. The inner rubber plug includes a lower inner rubber plug 82' and an upper inner rubber plug 83'. The shape of the end, where the sample absorption section of the test paper 3' is located, of the test paper cassette is adapted to the outer wall of the reagent cap 4', and the outer contour of the elastic sealing member is adapted to the inner cavity of the reagent cap 4'.

When the test paper 3' is to be mounted in the test paper cassette, the test paper 3' is firstly fixed to the lower inner rubber plug 82', then the upper inner rubber plug 83' and the lower inner rubber plug 82' are fixed, and it is guaranteed that the test paper 3' is fixed through interference fit between a groove of the lower inner rubber plug 82' and a rivet of the upper inner rubber plug 83' and interference fit between a rivet of the lower inner rubber plug 82' and a groove of the upper inner rubber plug 83'. The tail end of the test paper 3' is inserted into the outer rubber plug 84', the combined inner rubber plug is embedded with the outer rubber plug 84' by means of rubber material characteristics, the combined inner rubber plug and the outer rubber plug 84' are tightly matched. Then the above components are arranged in the upper cover 2' and the lower base 1' in an upper alignment mode to be clamped, and the upper cover 2' and the lower base 1' are completely sealed through the snap fit cooperation of the two positioning fasteners 8.

A certain amount of liquid can be sub-packaged in the reagent cap 4' in advance, and the reagent cap 4' is sealed with a sealing film or sealed by a cap, so that it is guaranteed that liquid in the device does not leak before use.

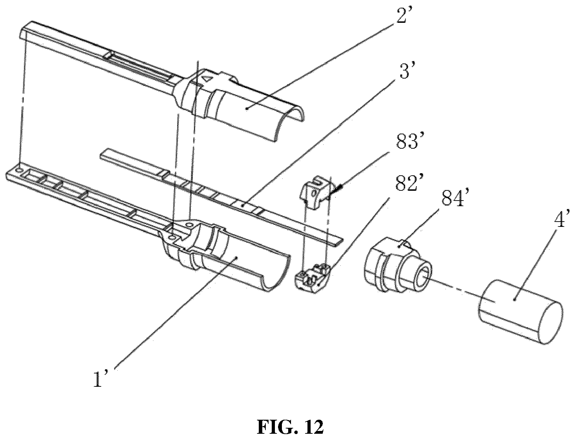

As shown in FIG. 11 and FIG. 12, when detection is needed, a sealing member of the reagent cap 4' is firstly opened, the test paper cassette is vertically inserted into the reagent cap 4' with the sample absorption section of the test paper 3' downwards until the test paper cassette is not pushed. It is guaranteed that the reagent bearing device is free of a liquid leakage risk through interference fit between the outer rubber plug 84' in the test paper cassette and the large-to-small inner cavity of the reagent cap 4' by means of rubber properties simultaneously. In addition, the tail ends of the upper cover 2' and the lower base 1' are enclosed to form an insertion member, the shape of the insertion member is basically consistent with the outer cavity of the reagent cap 4', and the insertion member is in interference fit with the outer cavity of the reagent cap 4', so that it is guaranteed that the reagent cap 4' can be stably fixed in the test paper cassette, thereby avoiding biological pollution.

In addition, the sample absorption section of the test paper 3' can be immersed into liquid in the reagent cap 4'. Through a press-fit between the combined inner rubber plug and the test paper 3' as well as between the outer rubber plug and the test paper 3', the liquid in the reagent cap 4' is prevented from rushing to the test paper 3' at the moment of horizontal placement. The liquid in the reagent cap 4' can be gradually chromatographed from the sample absorption end of the test paper 3' to the result display area of the test paper 3' and then to the top end of the test paper 3' until the chromatographic reaction is finished. Finally, a value is read by inserting the device into the instrument or by observing the observation window 22' through the naked eyes.

After 10-15 minutes of the reaction is finished, the instrument obtains a signal value after scanning and interpreting the area of the observation window 22', analyzes the signal value and converts the signal value into a detection result, and gives whether pathogen infection exists or not, or whether pathogen infection exists or not is given out by observing the line color developing intensity of the test paper 3' in the area of the observation window 22' in combination with a result interpretation standard.

The reagent cap 4' serves as a sample pre-processing device or a sample processing device, and processed sample types include not only swab samples, but also samples such as feces, blood and urine. Furthermore, the auxiliary test paper detection device in the present application can be used for non-quantitative detection and also can be used for quantitative detection.

### Working principle:

During use, through the cooperation of the upper cover 2' and the lower base 1', the test paper 3' is stably and fixedly clamped in a mounting gap formed through the cooperation of the upper cover 2' and the lower base 1', the sample absorption section of the test paper 3' extends out from one end of the test paper cassette and is exposed. The result display area of the test paper 3' corresponds to the observation window 22' of the test paper cassette. A certain amount of liquid is prepackaged in the reagent cap 4' in advance; during detection, the sealing member of the reagent cap 4' is firstly opened, and then the test paper cassette is vertically inserted into the reagent cap 4' with the sample absorption section of the test paper 3' downwards until the test paper cassette is not pushed. After 10-15 minutes of the reaction is finished, the instrument obtains a signal value after scanning and interpreting the area of the observation window 22', analyzes the signal value and converts the signal value into a detection result, and gives give whether pathogen infection exists or not, or whether pathogen infection exists or not is given out by observing the line color developing intensity of the test paper 3' in the area of the observation window 22' in combination with a result interpretation standard.

### Embodiment 5

FIG. 1.1 and FIG. 1.2 describe a device in the present disclosure. An auxiliary sample collection and detection device includes a test paper cassette and a reagent bearing device 4.

The assembled test paper cassette includes a lower base 1, an upper cover 2, a test paper 3 and a clamping sleeve 5. An accommodating groove for fixing test paper is formed in the lower base 1, the test paper 3 is fixed in the test paper accommodating groove of the lower base 1 in an upper alignment mode. The test paper is clamped and fixed through the positioning blocking ribs to be prevented from sliding inwards and outwards. When the upper cover 2 and the lower base 1 are in snap fit, the first positioning fastener 1.1 at the end of the lower base is in interference fit with the second positioning fastener 2.1 at the end of the upper cover, so that the upper cover 2 and the lower base 1 are completely sealed. The clamping sleeve 5 is clamped at the convex parts of the upper cover 2 and the lower base 1 from bottom to top until the clamping sleeve 5 is not pushed. An upper cover tail end fastener 2.3 and a lower base tail end fastener 1.3 are enclosed to form a fastener which is basically consistent with the inner ring of the clamping sleeve in shape. When the clamping sleeve is sleeved to a preset station, the inner ring of the clamping sleeve 5 abuts against and is fixed with the upper cover tail end fastener 2.3 and the lower base tail end fastener 1.3, so as to guarantee for a second time that the upper cover 2 and the lower base 1 are completely sealed.

A certain amount of liquid can be sub-packaged in the reagent bearing device 4 in advance, and the reagent bearing device is thermally sealed with a sealing film or sealed by a cap, so that it is guaranteed that liquid in the device does not leak before use.

In the embodiment, the sealing member of the reagent bearing device 4 is firstly opened, the test paper cassette is vertically inserted into the reagent bearing device 4 with the sample absorption section downwards until the test paper cassette is not pushed. The large-to-small structure of the inner ring of the reagent bearing device 4 is in interference fit with the small-to-large structure of the middle section 5.2 of the inner ring of the clamping sleeve. Therefore, when the reagent bearing device 4 is horizontally placed, liquid in the reagent bearing device 4 cannot leak into the clamping sleeve 5. Furthermore, the sample absorption section of the test paper 3 can be immersed into liquid of the reagent bearing device 4. The test paper 3 can be firmly fixed under the compression action on the test paper 3 by an upper cover positioning blocking rib 2.6 and a lower base positioning blocking rib 1.6, such compressing action also can prevent the liquid in the reagent bearing device 4 from rushing to the test paper 3 instantly when the detection device is horizontally placed. In 10-15 minutes of a chromatographic reaction, the liquid can be gradually chromatographed from the sample absorption end of the test paper 3 to a reaction window 2.2 and then to the top end of the test paper 3 until the chromatographic reaction is finished; then, a value is read by inserting the test paper into an instrument or by naked eyes.

Referring to FIG. 1.2, a reaction window 2.2 and a ventilated window 2.4 are formed in the upper end of the upper cover 2'. After 10-15 minutes of the reaction is finished, the instrument obtains a signal value after scanning and interpreting the area of the reaction window 2.2, analyzes the signal value and converts the signal value into a detection result, and gives whether pathogen infection exists or not, or whether pathogen infection exists or not is given out by observing the line color developing intensity of the test paper in the area of the reaction window 2.2 in combination with a result interpretation standard.

The reagent bearing device 4 serves as a sample preprocess device or a sample processing device, and processed sample types include not only swab samples, but also samples such as feces, blood and urine.

### Embodiment 6

FIG. 2.1 and FIG. 2.2 describe a device in the present disclosure. An auxiliary sample collection and detection device includes a test paper cassette and a reagent bearing device 4.

The assembled test paper cassette includes a lower base 1, an upper cover 2 and a test paper 3. An accommodating groove for fixing test paper is formed in the lower base 1, the test paper 3 is fixed in the test paper accommodating groove of the lower base 1 in an upper alignment mode to be clamped. When the upper cover 2 and the lower base 1 are in snap fit, the first positioning fastener 1.1 at the end of the lower base is in interference fit with the second positioning fastener 2.1 at the end of the upper cover, so that the upper cover 2 and the lower base 1 are completely sealed.

A certain amount of liquid can be sub-packaged in the reagent bearing device 4 in advance, and the reagent bearing device is thermally sealed with a sealing film or sealed by a cap, so that it is guaranteed that liquid in the device does not leak before use.

In the embodiment, a sealing member of the reagent bearing device 4 is firstly opened, and the test paper cassette is vertically inserted into the reagent bearing device 4 with the sample absorption section of the test paper downwards until the test paper cassette is not pushed. An upper cover tail end 2.31 and a lower base tail end 1.31 are enclosed to form an insertion member which is gradually enlarged from right to left and is basically consistent with the inner ring of the inner cavity of the reagent bearing device 4 in shape, the insertion member and the inner ring of the inner cavity of the reagent bearing device are in interference fit. Furthermore, a lower base handle 1.7 can perform secondary extrusion sealing on the reagent bearing device 4 at the same time, which can guarantee that the liquid in the reagent bearing device 4 cannot overflow in the reaction process, thereby avoiding the biological pollution. In addition, the arc-shaped design of the lower base handle 1.7 conforms to manual function science and is convenient for people to grasp manually. In addition, the sample absorption section of the test paper 3 can be immersed into liquid of the reagent bearing device 4, through the press fit between the lower base tail end 1.31 and the test paper 3 as well as the press-fit between the second positioning fastener 2.1 at the end of the upper cover and the test paper 3, the liquid in the reagent bearing device 4 is prevented from rushing to the test paper 3 instantly when the detection device is horizontally placed. In 10-15 minutes of a chromatographic reaction, the liquid can be gradually chromatographed from the sample absorption end of the test paper 3 to a reaction window 2.2 and then to the top end of the test paper 3 until the chromatographic reaction is finished. A value is read by inserting the test paper into an instrument or by naked eyes.

Referring to FIG. 2.2, a reaction window 2.2 is formed in the upper end of the upper cover 2. After 10-15 minutes of the reaction is finished, the instrument obtains a signal value after scanning and interpreting the area of the reaction window 2.2, analyzes the signal value and converts the signal value into a detection result, and gives whether pathogen infection exists or not, or whether pathogen infection exists or not is given out by observing the line color developing intensity of the test paper in the area of the reaction window 2.2 through naked eyes in combination with a result interpretation standard.

The reagent bearing device 4 serves as a sample pre-processing device or a sample processing device, and processed sample types include not only swab samples, but also samples such as feces, blood and urine.

### Embodiment 7

FIG. 3.1 and FIG. 3.2 describe a device in the present disclosure. An auxiliary sample collection and detection device includes a test paper cassette and a reagent bearing device 4.

The assembled test paper cassette includes a lower base 1, an upper cover 2 and a test paper 3. An accommodating groove for fixing test paper is formed in the lower base 1, the test paper 3 is fixed in the test paper accommodating groove of the lower base 1 in an upper alignment mode to be clamped. When the upper cover 2 and the lower base 1 are in snap fit, the first positioning fastener 1.1 at the end of the lower base is in interference fit with the second positioning fastener 2.1 at the end of the upper cover, and meanwhile, the first positioning fastener 1.1 at the tail end of the lower base and the second positioning fastener 2.1 at the tail end of the upper cover are also in interference fit, so that the upper cover 2 and the lower base 1 are completely sealed based on the interference fit between the top portion and the tail portion of the cover.

A certain amount of liquid can be sub-packaged in the reagent bearing device 4 in advance, and the reagent bearing device 4 is sealed with a sealing film or sealed by a cap, so that it is guaranteed that liquid in the device does not leak before use.

In the embodiment, a sealing member of the reagent bearing device 4 is firstly opened, and the test paper cassette is vertically inserted into the reagent bearing device 4 with the sample absorption section of the test paper downwards until the test paper cassette is not pushed; an upper cover tail end 2.31 and a lower bottom tail end 1.31 are enclosed to form an insertion member which is gradually enlarged from right to left and is basically consistent with the large-to-small inner ring of the inner cavity of the reagent bearing device 4 in shape, the insertion member and the inner ring of the inner cavity of the reagent bearing device are in interference fit, so that liquid in the bearing device 4 can be guaranteed not to overflow in the reaction process, thereby avoiding biological pollution. In addition, the sample absorption section of the test paper 3 can be completely immersed into liquid of the reagent bearing device 4, through the press-fit cooperation between the lower base tail end 1.31 and the test paper 3 as well as the press-fit cooperation between the upper cover tail end 2.31 and the test paper 3, the liquid in the reagent bearing device 4 is prevented from rushing to the test paper 3 instantly when the detection device is horizontally placed. In 10-15 minutes of a chromatographic reaction, the liquid can be gradually chromatographed from the sample absorption end of the test paper 3 to a reaction window 2.2 and then to the top end of the test paper 3 until the chromatographic reaction is finished; then, a value can be read by inserting the test paper into an instrument or by naked eyes.

Referring to FIG. 3.1, a reaction window 2.2 is formed in the upper end of the upper cover 2. After 10-15 minutes of the reaction is finished, the instrument obtains a signal value after scanning and interpreting the area of the reaction window 2.2, analyzes the signal value and converts the signal value into a detection result, and gives whether pathogen infection exists or not, or whether pathogen infection exists or not is given out by observing the line color developing intensity of the test paper in the area of the reaction window 2.2 through naked eyes, in combination with a result interpretation standard.

The reagent bearing device 4 serves as a sample pre-processing device or a sample processing device, and processed sample types include not only swab samples, but also samples such as feces, blood and urine.

### Embodiment 8

FIG. 4.1, FIG. 4.2 and FIG. 3 describe a device in the present disclosure. An auxiliary sample collection and detection device includes a test paper cassette and a reagent bearing device 4.

The test paper cassette includes a lower base 1, an upper cover 2, a test paper 3, a lower inner rubber plug 5, an upper inner rubber plug 6 and an outer rubber plug 7. An accommodating groove for fixing test paper is formed in the lower base 1, the test paper 3 is firstly fixed to the lower inner rubber plug 5, then the upper inner rubber plug 6 and the lower inner rubber plug 5 are fixed, and it is guaranteed that the test paper 3 is fixed through interference fit between a lower-inner-rubber-plug groove 5.1 and an upper-inner-rubber-plug rivet 6.2 and interference fit between a lower-inner-rubber-plug rivet 5.2 and an upper-inner-rubber-plug groove 6.1. The tail end of the test paper 3 is inserted into the outer rubber plug 7, the combined inner rubber plug is embedded with the outer rubber plug 7 by means of rubber material characteristics, the combined inner rubber plug and the outer rubber plug 7 are tightly matched. The above components are arranged in the lower base 1 in an upper alignment mode to be clamped, the upper cover 2 and the lower base 1 are sealed. At the upper end of the detection device, the first positioning fastener 1.1 at the end part of the lower base is in interference fit with the second positioning fastener 2.1 at the end part of the upper cover. At the tail end of the detection device, the two first positioning fasteners 1.1 at the tail portion of the lower base are in interference fit with the second positioning fastener 2.1 at the tail portion of the upper cover, which can guarantee that the upper cover 2 and the lower base 1 can be completely sealed.

A certain amount of liquid can be sub-packaged in the reagent bearing device 4 in advance, and the reagent bearing device is sealed thermally with a sealing film or sealed by a cap, so that it is guaranteed that liquid in the device does not leak before use.

In the embodiment, a sealing member of the reagent bearing device 4 is firstly opened, and the test paper cassette is vertically inserted into the reagent bearing device 4 with the sample absorption section of the test paper downwards until the test paper cassette is not pushed. It is guaranteed that the reagent bearing device is free of a liquid leakage risk through interference fit between the outer rubber plug 7 in the test paper cassette and the large-to-small inner cavity of the reagent bearing device 4 by means of rubber properties simultaneously. The tail end of the upper cover 2 and the tail end of the lower base 1 are enclosed to form an insertion member which is basically consistent with the outer cavity of the reagent bearing device in shape, the insertion member and the outer cavity of the reagent bearing device are in interference fit, so that the reagent bearing device can be stably fixed in the test paper cassette, thereby avoiding biological pollution. In addition, the sample absorption section of the test paper 3 can be immersed into liquid of the reagent bearing device 4, through the press fit between the combined inner rubber plug and the test paper 3 as well as that between the outer rubber plug 7 and the test paper 3, the liquid in the reagent bearing device 4 is prevented from rushing to the test paper 3 instantly when the detection device is horizontally placed. In 10-15 minutes of a chromatographic reaction, the liquid can be gradually chromatographed from the sample absorption end of the test paper 3 to a reaction window 2.2 and then to the top end of the test paper 3 until the chromatographic reaction is finished. A value is read by inserting the test paper into an instrument or by naked eyes.

Referring to FIG. 4.1, a reaction window 2.2 is formed in the upper end of the upper cover 2. After 10-15 minutes of the reaction is finished, the instrument obtains a signal value after scanning and interpreting the area of the reaction window 2.2, analyzes the signal value and converts the signal value into a detection result, and gives whether pathogen infection exists or not, or whether pathogen infection exists or not is given out by observing the line color developing intensity of the test paper in the area of the reaction window 2.2 through naked eyes, in combination with a result interpretation standard.

The reagent bearing device 4 serves as a sample pre-processing device or a sample processing device, and processed sample types include not only swab samples, but also samples such as feces, blood and urine.

Several examples are used for illustration of the principles and implementation methods of the present disclosure. The description of the embodiments is used to help illustrate the method and its core principles of the present disclosure. In addition, those skilled in the art can make various modifications in terms of specific embodiments and scope of application in accordance with the teachings of the present disclosure. In conclusion, the content of this specification shall not be construed as a limitation to the present disclosure.

## Claims

1. An auxiliary test paper detection device, comprising a reagent cap (4') and a test paper cassette, wherein the reagent cap (4') is configured for containing a detection reagent, a sealing member is arranged at an opening of the reagent cap (4'), and the sealing member is opened or removed under action of external force;
a test paper (3') is arranged in the test paper cassette, a sample absorption section of the test paper (3') is exposed, an observation window (22') is formed in the test paper cassette, and a result display area of the test paper (3') corresponds to the observation window (22'); and
a connecting structure for connecting the test paper cassette and the reagent cap (4') is provided; when the test paper cassette and the reagent cap (4') are connected through the connecting structure, a closed space is formed by an inner cavity of the reagent cap (4'), and the sample absorption section of the test paper (3') extends into the inner cavity of the reagent cap (4').

2. The auxiliary test paper detection device according to claim 1, wherein the test paper cassette comprises an upper cover (2') and a lower base (1') which are in snap fit with each other, a gap for accommodating the test paper (3') is formed between the upper cover (2') and the lower base (1'), and the upper cover (2') and the lower base (1') each are provided with positioning fasteners (5') and first positioning blocking ribs (51'); and
when the upper cover (2') and the lower base (1') are in snap fit, the positioning fasteners (5') on the upper cover (2') and the lower base (1') are in snap fit with each other to form positioning lock, and the first positioning blocking ribs (51') on the upper cover (2') and the lower base (1') cooperatively and tightly abut against the test paper (3').

3. The auxiliary test paper detection device according to claim 2, wherein the connecting structure comprises a connecting clamping sleeve (52') and tail end fasteners (53'), an inner periphery of an inner ring of the connecting clamping sleeve (52') is fixedly sleeved on the test paper cassette, and an outer periphery of the inner ring of the connecting clamping sleeve (52') is sleeved with the reagent cap and in interference fit with the reagent cap (4');
the tail end fasteners (53') are elastic, and the tail end fasteners (53') are respectively arranged on the upper cover (2') and the lower base (1') at an end of the test paper cassette, fixedly sleeved with the connecting clamping sleeve (52'); and
when the connecting clamping sleeve (52') is sleeved to a predetermined station, the inner ring of the connecting clamping sleeve (52') is in snap fit with the tail end fasteners (53') on the upper cover (2') and the lower base (1').

4. The auxiliary test paper detection device according to claim 1, wherein the test paper cassette comprises an upper cover (2') and a lower base (1'), a positioning hole (6') is formed in the upper cover (2'), a positioning convex block (61') is arranged on the lower base (1'), the positioning convex block (61') is embedded in the positioning hole (6') and in interference fit with the positioning holes (6'); a gap for accommodating the test paper (3') is formed between the upper cover (2') and the lower base (1'); and an end of the test paper cassette, from which the sample absorption section of the test paper (3') extends out, has a shape adapted to an inner wall of the reagent cap (4').

5. The auxiliary test paper detection device according to claim 4, wherein the connecting structure comprises two handles (62'), the two handles (62') are respectively and movably arranged at two sides of the end of the test paper cassette, from which the sample absorption section of the test paper (3') extends out, and main bodies of the two handles (62') extend outwards in an insertion direction of the reagent cap (4').

6. The auxiliary test paper detection device according to claim 1, wherein the test paper cassette comprises an upper cover (2') and a lower base (1') which are in snap fit with each other, a gap for accommodating the test paper (3') is formed between the upper cover (2') and the lower base (1'), positioning mounting holes (7') are formed in the upper cover (2'), and mounting columns (71') are arranged on the lower base (1'); and
when the upper cover (2') and the lower base (1') are in snap fit with each other, the mounting columns (71') are inserted into the positioning mounting holes (7') and in interference fit with the positioning mounting holes (7').

7. The auxiliary test paper detection device according to claim 6, wherein the connecting structure comprises an insertion member (72'), an outer contour of the insertion member (72') corresponds to a side wall of an inner cavity of the reagent cap (4'), and the insertion member (72') is in interference fit with the side wall of the inner cavity of the reagent cap (4').

8. The auxiliary test paper detection device according to claim 1, wherein the test paper cassette comprises an upper cover (2') and a lower base (1') which are in snap fit with each other, a gap for accommodating the test paper (3') is formed between the upper cover (2') and the lower base (1'), positioning fasteners (8') are arranged on the upper cover (2') and the lower base (1'), and second positioning blocking ribs (81') are arranged on the upper cover and the lower base; and
when the upper cover (2') is in snap fit with the lower base (1'), the positioning fasteners (8') on the upper cover (2') and the lower base (1') are in snap fit together and form a positioning lock, and the second positioning blocking ribs (81') on the upper cover (2') and the lower base (1') cooperatively and tightly abut against the test paper (3').

9. The auxiliary test paper detection device according to claim 8, wherein the connecting structure comprises an elastic sealing member, the elastic sealing member is arranged between the test paper (3') and the test paper cassette, and the elastic sealing member is located at an end of the test pater cassette where the sample absorption section of the test paper (3') is located; and
the end of the test pater cassette where the sample absorption section of the test paper (3') is located has a shape adapted to an outer wall of the reagent cap (4'), and an outer contour of the elastic sealing member is adapted to the inner cavity of the reagent cap (4').

10. The auxiliary test paper detection device according to claim 9, wherein the elastic sealing member comprises inner rubber plugs (83') and an outer rubber plug (84'), the inner rubber plugs (83') comprise a pair of split rubber plugs which are connected with each other in an insertion manner, the inner rubber plugs (83') are sleeved on the test paper (3'), one end of the outer rubber plug (84') is sleeved on the inner rubber plugs (83') to form secondary sealing, and another end of the outer rubber plug (84') is inserted into the inner cavity of the reagent cap (4') to form sealing.

## Patentansprüche

1. Hilfstestpapier-Nachweisvorrichtung, die eine Reagenzienkappe (4') und eine Testpapierkassette umfasst, wobei die Reagenzienkappe (4') so konfiguriert ist, dass sie ein Nachweisreagenz enthält, ein Verschlusselement an einer Öffnung der Reagenzienkappe (4') angeordnet ist und das Verschlusselement unter Einwirkung einer äußeren Kraft geöffnet oder entfernt wird;
ein Testpapier (3') ist in der Testpapierkassette angeordnet, ein Probenabsorptionsabschnitt des Testpapiers (3') ist freigelegt, ein Beobachtungsfenster (22') ist in der Testpapierkassette geformt und ein Ergebnisanzeigebereich des Testpapiers (3') entspricht dem Beobachtungsfenster (22'); und
eine Verbindungsstruktur zum Verbinden der Testpapierkassette und der Reagenzienkappe (4') ist vorgesehen; wenn die Testpapierkassette und die Reagenzienkappe (4') durch die Verbindungsstruktur verbunden werden, wird ein geschlossener Raum durch einen inneren Hohlraum der Reagenzienkappe (4') gebildet, und der Probenabsorptionsabschnitt des Testpapiers (3') erstreckt sich in den inneren Hohlraum der Reagenzienkappe (4').

2. Hilfstestpapier-Nachweisvorrichtung nach Anspruch 1, wobei die Testpapierkassette eine obere Abdeckung (2') und eine untere Basis (1') umfasst, die in Schnapppassung miteinander sind, wobei ein Spalt zur Aufnahme des Testpapiers (3') zwischen der oberen Abdeckung (2') und der unteren Basis (1') gebildet ist, und wobei die obere Abdeckung (2') und die untere Basis (1') jeweils mit Positionierungsverschlüssen (5') und ersten Positionierungssperrrippen (51') versehen sind; und
wenn die obere Abdeckung (2') und die untere Basis (1') in Schnapppassung sind, sind die Positionierungsverschlüsse (5') an der oberen Abdeckung (2') und der unteren Basis (1') in Schnapppassung miteinander, um eine Positionierungsverriegelung zu bilden, und die ersten Positionierungssperrrippen (51') an der oberen Abdeckung (2') und der unteren Basis (1') liegen zusammenwirkend und dicht gegen das Testpapier (3') an.

3. Hilfstestpapier-Nachweisvorrichtung nach Anspruch 2, wobei die Verbindungsstruktur eine Verbindungsklemmhülse (52') und Endbefestigungselemente (53') umfasst, wobei ein innerer Umfang eines inneren Rings der Verbindungsklemmhülse (52') fest auf die Testpapierkassette aufgeschoben ist, und ein äußerer Umfang des inneren Rings der Verbindungsklemmhülse (52') mit der Reagenzienkappe aufgeschoben ist und in Presspassung mit der Reagenzienkappe (4') ist;
wobei die Endbefestigungselemente (53') elastisch sind und die Endbefestigungselemente (53') jeweils an der oberen Abdeckung (2') und der unteren Basis (1') an einem Ende der Testpapierkassette angeordnet sind und fest mit der Verbindungsklemmhülse (52') ummantelt sind; und
wenn die Verbindungsklemmhülse (52') zu einer vorbestimmten Station geschoben wird, ist der Innenring der Verbindungsklemmhülse (52') in Schnappsitz mit den Endbefestigungselementen (53') an der oberen Abdeckung (2') und der unteren Basis (1').

4. Hilfstestpapier-Nachweisvorrichtung nach Anspruch 1, wobei die Testpapierkassette eine obere Abdeckung (2') und eine untere Basis (1') umfasst, ein Positionierungsloch (6') ist in der oberen Abdeckung (2') ausgebildet, ein konvexer Positionierungsblock (61') ist an der unteren Basis (1') angeordnet, der konvexe Positionierungsblock (61') ist in das Positionierungsloch (6') eingebettet und ist in Presspassung mit den Positionierungslöchern (6'); ein Spalt zur Aufnahme des Testpapiers (3') ist zwischen der oberen Abdeckung (2') und der unteren Basis (1') ausgebildet; und ein Ende der Testpapierkassette, aus dem der Probenaufnahmeabschnitt des Testpapiers (3') herausragt, weist eine an eine Innenwand der Reagenzienkappe (4') angepasste Form auf.

5. Hilfstestpapier-Nachweisvorrichtung nach Anspruch 4, wobei die Verbindungsstruktur zwei Griffe (62') umfasst, die beiden Griffe (62') jeweils beweglich an zwei Seiten des Endes der Testpapierkassette angeordnet sind, aus der der Probenabsorptionsabschnitt des Testpapiers (3') herausragt, und sich die Hauptkörper der beiden Griffe (62') in einer Einführrichtung der Reagenzienkappe (4') nach außen erstrecken.

6. Hilfstestpapier-Nachweisvorrichtung nach Anspruch 1, wobei die Testpapierkassette eine obere Abdeckung (2') und eine untere Basis (1') umfasst, die in Schnapppassung miteinander sind, ein Spalt zur Aufnahme des Testpapiers (3') ist zwischen der oberen Abdeckung (2') und der unteren Basis (1') gebildet, Positionierungs-Befestigungslöcher (7') sind in der oberen Abdeckung (2') gebildet und Befestigungssäulen (71') sind an der unteren Basis (1') angeordnet; und
wenn die obere Abdeckung (2') und die untere Basis (1') in Schnapppassung miteinander sind, werden die Befestigungssäulen (71') in die Positionierungsbefestigungslöcher (7') eingesetzt und sind in Presspassung mit den Positionierungsbefestigungslöchern (7').

7. Hilfstestpapier-Nachweisvorrichtung nach Anspruch 6, wobei die Verbindungsstruktur ein Einführungselement (72') umfasst, eine Außenkontur des Einführungselements (72') einer Seitenwand eines inneren Hohlraums der Reagenzienkappe (4') entspricht, und das Einführungselement (72') in Presspassung mit der Seitenwand des inneren Hohlraums der Reagenzienkappe (4') ist.

8. Hilfstestpapier-Nachweisvorrichtung nach Anspruch 1, wobei die Testpapierkassette eine obere Abdeckung (2') und eine untere Basis (1') umfasst, die in Schnapppassung miteinander sind, wobei ein Spalt zur Aufnahme des Testpapiers (3') zwischen der oberen Abdeckung (2') und der unteren Basis (1') gebildet ist, wobei Positionierungsverschlüsse (8') an der oberen Abdeckung (2') und der unteren Basis (1') angeordnet sind, und wobei zweite Positionierungssperrrippen (81') an der oberen Abdeckung und der unteren Basis angeordnet sind; und
wenn die obere Abdeckung (2') in Schnapppassung mit der unteren Basis (1') ist, sind die Positionierungsbefestigungselemente (8') an der oberen Abdeckung (2') und der unteren Basis (1') in Schnapppassung miteinander und bilden eine Positionierungsverriegelung, und die zweiten Positionierungssperrrippen (81') an der oberen Abdeckung (2') und der unteren Basis (1') liegen zusammenwirkend und dicht gegen das Testpapier (3') an.

9. Hilfstestpapier-Nachweisvorrichtung nach Anspruch 8, wobei die Verbindungsstruktur ein elastisches Dichtungselement umfasst, das elastische Dichtungselement zwischen dem Testpapier (3') und der Testpapierkassette angeordnet ist und das elastische Dichtungselement an einem Ende der Testpapierkassette angeordnet ist, an dem sich der Probenaufnahmeabschnitt des Testpapiers (3') befindet; und
das Ende der Testpapierkassette, an dem sich der Probenaufnahmeabschnitt des Testpapiers (3') befindet, eine Form aufweist, die an eine Außenwand der Reagenzienkappe (4') angepasst ist, und eine Außenkontur des elastischen Dichtungselements an den inneren Hohlraum der Reagenzienkappe (4') angepasst ist.

10. Hilfstestpapier-Nachweisvorrichtung nach Anspruch 9, wobei das elastische Dichtungselement innere Gummistopfen (83') und einen äußeren Gummistopfen (84') umfasst, die inneren Gummistopfen (83') ein Paar geteilter Gummistopfen umfassen, die miteinander in einer Einschubart verbunden sind, die inneren Gummistopfen (83') auf das Testpapier (3') aufgeschoben sind, ein Ende des äußeren Gummistopfens (84') auf die inneren Gummistopfen (83') aufgeschoben ist, um eine Sekundärdichtung zu bilden, und ein anderes Ende des äußeren Gummistopfens (84') in den inneren Hohlraum der Reagenzienkappe (4') eingeführt ist, um eine Dichtung zu bilden.

## Revendications

1. Dispositif auxiliaire de détection de papier d'essai, comprenant un capuchon de réactif (4') et une cassette de papier d'essai, dans lequel le capuchon de réactif (4') est configuré pour contenir un réactif de détection, un élément d'étanchéité est agencé au niveau d'une ouverture du capuchon de réactif (4'), et l'élément d'étanchéité est ouvert ou retiré sous l'action d'une force externe ;
un papier d'essai (3') est agencé dans la cassette de papier d'essai, une section d'absorption d'échantillon du papier d'essai (3') est exposée, une fenêtre d'observation (22') est formée dans la cassette de papier d'essai, et une zone d'affichage de résultat du papier d'essai (3') correspond à la fenêtre d'observation (22') ; et
une structure de liaison pour relier la cassette de papier d'essai et le capuchon de réactif (4') est prévue ; lorsque la cassette de papier d'essai et le capuchon de réactif (4') sont reliés par le biais de la structure de liaison, un espace fermé est formé par une cavité intérieure du capuchon de réactif (4'), et la section d'absorption d'échantillon du papier d'essai (3') s'étend dans la cavité intérieure du capuchon de réactif (4').

2. Dispositif auxiliaire de détection de papier d'essai selon la revendication 1, dans lequel la cassette de papier d'essai comprend un couvercle supérieur (2') et une base inférieure (1') qui sont emboîtés l'un avec l'autre, un vide destiné à recevoir le papier d'essai (3') est formé entre le couvercle supérieur (2') et la base inférieure (1'), et le couvercle supérieur (2') et la base inférieure (1') sont chacun pourvus d'éléments de fixation de positionnement (5') et de premières nervures de blocage de positionnement (51') ; et
lorsque le couvercle supérieur (2') et la base inférieure (1') sont emboîtés, les éléments de fixation de positionnement (5') sur le couvercle supérieur (2') et la base inférieure (1') sont emboîtés les uns avec les autres pour former un verrou de positionnement, et les premières nervures de blocage de positionnement (51') sur le couvercle supérieur (2') et la base inférieure (1') viennent buter contre le papier d'essai (3') de manière coopérative et étroite.

3. Dispositif auxiliaire de détection de papier d'essai selon la revendication 2, dans lequel la structure de liaison comprend un manchon de serrage de liaison (52') et des éléments de fixation d'extrémité de queue (53'), une périphérie intérieure d'une bague intérieure du manchon de serrage de liaison (52') est emmanchée à demeure sur la cassette de papier d'essai, et une périphérie extérieure de la bague intérieure du manchon de serrage de liaison (52') est emmanchée avec le capuchon de réactif et en ajustement avec serrage avec le capuchon de réactif (4') ;
les éléments de fixation d'extrémité de queue (53') sont élastiques, et les éléments de fixation d'extrémité de queue (53') sont respectivement agencés sur le couvercle supérieur (2') et la base inférieure (1') au niveau d'une extrémité de la cassette de papier d'essai, emmanchée à demeure avec le manchon de serrage de liaison (52') ; et
lorsque le manchon de serrage de liaison (52') est emmanché jusqu'à une station prédéterminée, la bague intérieure du manchon de serrage de liaison (52') est en ajustement avec serrage avec les éléments de fixation d'extrémité de queue (53') sur le couvercle supérieur (2') et la base inférieure (1').

4. Dispositif auxiliaire de détection de papier d'essai selon la revendication 1, dans lequel la cassette de papier d'essai comprend un couvercle supérieur (2') et une base inférieure (1'), un trou de positionnement (6') est formé dans le couvercle supérieur (2'), un bloc convexe de positionnement (61') est agencé sur la base inférieure (1'), le bloc convexe de positionnement (61') est encastré dans le trou de positionnement (6') et en ajustement avec serrage avec les trous de positionnement (6') ; un vide destiné à recevoir le papier d'essai (3') est formé entre le couvercle supérieur (2') et la base inférieure (1') ; et une extrémité de la cassette de papier d'essai, par laquelle la section d'absorption d'échantillon du papier d'essai (3') s'étend, présente une forme adaptée à une paroi intérieure du capuchon de réactif (4').

5. Dispositif auxiliaire de détection de papier d'essai selon la revendication 4, dans lequel la structure de liaison comprend deux poignées (62'), les deux poignées (62') sont agencées de façon respective et mobile au niveau de deux côtés de l'extrémité de la cassette de papier d'essai, par laquelle la section d'absorption d'échantillon du papier d'essai (3') s'étend, et des corps principaux des deux poignées (62') s'étendent vers l'extérieur dans une direction d'insertion du capuchon de réactif (4').

6. Dispositif auxiliaire de détection de papier d'essai selon la revendication 1, dans lequel la cassette de papier d'essai comprend un couvercle supérieur (2') et une base inférieure (1') qui sont emboîtés l'un avec l'autre, un vide destiné à recevoir le papier d'essai (3') est formé entre le couvercle supérieur (2') et la base inférieure (1'), des trous de montage de positionnement (7') sont formés dans le couvercle supérieur (2') et des colonnes de montage (71') sont agencées sur la base inférieure (1') ; et
lorsque le couvercle supérieur (2') et la base inférieure (1') sont emboîtés l'un avec l'autre, les colonnes de montage (71') sont insérées dans les trous de montage de positionnement (7') et en ajustement avec serrage avec les trous de montage de positionnement (7').

7. Dispositif auxiliaire de détection de papier d'essai selon la revendication 6, dans lequel la structure de liaison comprend un élément d'insertion (72'), un contour extérieur de l'élément d'insertion (72') correspond à une paroi latérale d'une cavité intérieure du capuchon de réactif (4'), et l'élément d'insertion (72') est en ajustement avec serrage avec la paroi latérale de la cavité intérieure du capuchon de réactif (4').

8. Dispositif auxiliaire de détection de papier d'essai selon la revendication 1, dans lequel la cassette de papier d'essai comprend un couvercle supérieur (2') et une base inférieure (1') qui sont emboîtés l'un avec l'autre, un vide destiné à recevoir le papier d'essai (3') est formé entre le couvercle supérieur (2') et la base inférieure (1'), des éléments de positionnement (8') sont agencés sur le couvercle supérieur (2') et le base inférieure (1'), et des secondes nervures de blocage de positionnement (81') sont agencées sur le couvercle supérieur et la base inférieure ; et
lorsque le couvercle supérieur (2') est emboîté avec le base inférieure (1'), les éléments de fixation de positionnement (8') sur le couvercle supérieur (2') et la base inférieure (1') sont emboîtés entre eux et forment un verrou de positionnement, et les secondes nervures de blocage de positionnement (81') sur le couvercle supérieur (2') et la base inférieure (1') viennent buter contre le papier d'essai (3') de manière coopérative et étroite.

9. Dispositif auxiliaire de détection de papier d'essai selon la revendication 8, dans lequel la structure de liaison comprend un élément d'étanchéité élastique, l'élément d'étanchéité élastique est agencé entre le papier d'essai (3') et la cassette de papier d'essai, et l'élément d'étanchéité élastique est situé au niveau d'une extrémité de la cassette de papier d'essai où la section d'absorption d'échantillon du papier d'essai (3') est située ; et
l'extrémité de la cassette de papier d'essai où la section d'absorption d'échantillon du papier d'essai (3') est située présente une forme adaptée à une paroi extérieure du capuchon de réactif (4'), et un contour extérieur de l'élément d'étanchéité élastique est adapté à la cavité intérieure du capuchon de réactif (4').

10. Dispositif auxiliaire de détection de papier d'essai selon la revendication 9, dans lequel l'élément d'étanchéité élastique comprend des bouchons en caoutchouc intérieurs (83') et un bouchon en caoutchouc extérieur (84'), les bouchons en caoutchouc intérieurs (83') comprennent une paire de bouchons en caoutchouc fendus qui sont reliés l'un à l'autre par insertion, les bouchons en caoutchouc intérieurs (83') sont emmanchés sur le papier d'essai (3'), une extrémité du bouchon en caoutchouc extérieur (84') est emmanchée sur les bouchons en caoutchouc intérieurs (83') pour former une étanchéité secondaire, et une autre extrémité du bouchon en caoutchouc extérieur (84') est insérée dans la cavité intérieure du capuchon de réactif (4') pour former une étanchéité.
